(19) Europäisches Patentamt / European Patent Office / Office européen des brevets

(11) **EP 3 314 244 B1**

(12) **EUROPEAN PATENT SPECIFICATION**

(45) Date of publication and mention of the grant of the patent:
**08.05.2019  Bulletin 2019/19**

(51) Int Cl.:
***G01N 27/22*** *(2006.01)*    ***G01N 33/44*** *(2006.01)*

(21) Application number: **16751365.4**

(22) Date of filing: **22.06.2016**

(86) International application number:
**PCT/NL2016/050441**

(87) International publication number:
**WO 2016/209072 (29.12.2016 Gazette 2016/52)**

(54) **MEASURING DEVICE FOR MEASURING CHANGE IN DIELECTRIC PROPERTIES IN AN ENVIRONMENT WITH VARIABLE DIELECTRIC PROPERTIES**

MESSVORRICHTUNG ZUR MESSUNG VON VERÄNDERUNGEN DIELEKTRISCHER EIGENSCHAFTEN IN EINER UMGEBUNG MIT VARIABLEN DIELEKTRISCHEN EIGENSCHAFTEN

DISPOSITIF DE MESURE POUR MESURER UN CHANGEMENT DANS LES PROPRIÉTÉS DIÉLECTRIQUES DANS UN ENVIRONNEMENT À PROPRIÉTÉS DIÉLECTRIQUES VARIABLES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **25.06.2015   NL 2015028**

(43) Date of publication of application:
**02.05.2018   Bulletin 2018/18**

(73) Proprietor: **Kooyen Holding B.V.**
**7683 RR Den Ham (NL)**

(72) Inventors:
 • **BOKSEM, Cornelis**
   **7683 RR Den Ham (NL)**
 • **VAN DER HEIDE, Frank Bastiaan**
   **7683 CK Den Ham (NL)**
 • **LEEMKUIL, Gerhardus Matheus Plechelmus**
   **7557 XE Hengelo (NL)**

(74) Representative: **Mink-Lindenburg, Charlotte Hildegard**
**Octrooibureau Mink B.V.**
**P.O. Box 85658**
**2508 CJ The Hague (NL)**

(56) References cited:
**EP-A2- 0 922 963**

 • **Huan L Lee: "The Handbook of Dielectric Analysis and Cure Monitoring", , 2014, XP055250111, Boston, USA Retrieved from the Internet: URL:http://www.lambient.com/docs/DielectricAnalysisCureMonitoringHandbook.pdf [retrieved on 2016-02-15]**

**Description**

The present invention relates to a measuring device for measuring change in the dielectric properties of an environment, comprising

- a capacitive measuring circuit provided with an input and output electrical connection, wherein the measuring circuit comprises one or more coupled measuring capacitors which are placeable in the environment and wherein the capacitance of each of the measuring capacitors can be influenced by the dielectric properties of the environment,
- an electric alternating signal generator for applying a first electric alternating signal with a specific base frequency and a specific signal shape between the input and output connection of the measuring circuit,
- a first signal processing means for deriving a second electric alternating signal from the first signal, wherein the second signal has the same base frequency and signal shape as the first signal and is shifted 90° in phase relative to the first signal,
- first quadrature determining means for calculating the in-phase component $I1_C$ and quadrature component $Q1_C$ of an output voltage Uc across the output connection of the measuring circuit, wherein the first signal and second signal function as reference signals.

A measuring device according to the preamble is known in the field. The known measuring device is described in the European patent application EP 0922963.

A device for monitoring curing of a resin by measuring changes in its dielectric properties is known from Huan L Lee: "The Handbook of Dielectric Analysis and Cure Monitoring", 2014, XP055250111, Boston, USA.

The known measuring device is applied for the purpose of analysing the properties of liquids, such as for instance to determine the ageing of lubricating oils or moisture measurement in the ground.

The magnitude of the output voltage is defined by the formula:

$$Magnitude = \sqrt[2]{I1_L^2 + Q1_L^2}$$

The change in magnitude of the output voltage Uc is a measure of the change in the capacitance of the measuring circuit, and thereby in the properties of liquids.

The known measuring device has the drawback that in many cases the measuring device is not sufficiently accurate. Small changes in the dielectric properties of the environment result in a very small change in the in-phase component $I1_C$ and quadrature component $Q1_C$, whereby the known measuring device is not accurate enough for many applications.

An example of an application for which the known measuring device is not suitable is the measurement of changes in synthetic resin in a resin infusion process as applied in the manufacture of a blade of a wind turbine. The changes in the dielectric properties of synthetic resin are very small, whereby the known measuring device misses subtle changes in the production process.

It is an object of the invention to provide a measuring device which is much more accurate than the known measuring device.

The invention provides for this purpose a device according to the preamble, characterized in that the measuring device is provided with

- second quadrature determining means for calculating the in-phase component $I1_L$ and quadrature component $Q1_L$ of an input voltage $U_L$ across the input connection of the measuring circuit, wherein the first signal and second signal function as reference signals,
- a calculation circuit configured to calculate a ratio of the in-phase components ($I1_L$, $I1_C$) and quadrature components ($Q1_L$, $Q1_C$) of the output voltage ($U_L$) and input voltage ($U_C$).

The invention is based on the insight that the in-phase component and quadrature component of the output voltage are not only influenced by the measuring circuit, but that the measuring circuit itself also influences the input voltage. By determining said ratio only the influence of the measuring circuit, i.e. the change in the capacitance of the measuring capacitors, is determined.

In a first embodiment of the measuring device according to the invention the following formula is determinant for calculating the ratio:

$$RATIO = \sqrt[2]{\frac{I1_L^2 + Q1_L^2}{I1_C^2 + Q1_C^2}}$$

The ratio calculates the relation between the magnitudes of the vectors in the phasor diagram of the input and output voltage. The change in the capacitance of the measuring capacitors resulting from the change in the dielectric can hereby be tracked in advantageous manner.

In order to increase the dynamic range of the calculated ratio, in a second embodiment of the measuring device according to the invention the signal generator is provided with

- a second signal processing means for deriving a third electric alternating signal from the first signal, wherein the third signal has the same base frequency and signal shape as the first signal and is shifted 180° in phase relative to the first signal,
- a third signal processing means for deriving a fourth electric alternating signal from the first signal, wherein the fourth signal has the same base frequency and signal shape as the first signal and is shifted 270° in phase relative to the first signal,
- third quadrature determining means for calculating the in-phase component $I2_C$ and quadrature component Q2c of an output voltage Uc across the output connection of the measuring circuit, wherein the third signal and fourth signal function as reference signals,
- fourth quadrature determining means for calculating the in-phase component $I2_L$ and quadrature component $Q2_L$ of an input voltage $U_L$ across the input connection of the measuring circuit, wherein the third signal and fourth signal function as reference signals,

wherein the calculation circuit is configured to calculate a ratio of the in-phase components ($I1_L$, $I2_L$, $I1_C$, $I2_C$) and quadrature components ($Q1_L$, $Q2_L$, $Q1_C$, $Q2_C$) of the output voltage ($U_L$) and input voltage ($U_C$).

As a result of these measures the cosine component of the input and output voltage is included as well as the sine component in the calculation of the ratio.

In this second embodiment the following formula is preferably determinant for calculating the ratio:

$$RATIO = \sqrt[2]{\frac{(|I1_L|+|I2_L|)^2 + (|Q1_L|+|Q2_L|)^2}{(|I1_C|+|I2_C|)^2 + (|Q1_C|+|Q2_C|)^2}}$$

The calculation circuit of the measuring device according to the invention preferably comprises one or more analog to digital converters for converting the analog in-phase signals and analog quadrature signals to digital in-phase and digital quadrature signals, and one or more programmed circuits for calculating the ratio with the digital in-phase and quadrature signals.

In order to better track the changes in the ratio, the calculation circuit of the measuring device according to the invention is preferably also configured to calculate a differential quotient of the ratio.

In order to also track the overall change in the ratio in a specific interval the calculation circuit of the measuring device is preferably also configured to calculate an integral of the ratio over a fixed time interval.

In order to enable comparison to previous measurements, the measuring device according to the invention preferably comprises storage means for storing a ratio, differential of the ratio and/or integral of the ratio in relation to time during a measurement, and comparing means for comparing a ratio to a stored ratio.

All computational operations for calculating the ratio, differential quotient of the ratio and/or integral of the ratio are preferably performed in a programmable unit. The calculation circuit of the measuring device according to the invention preferably comprises for this purpose a computer, wherein the computer is configured to calculate data relating to the ratio, the differential quotient of the ratio and/or the integral of the ratio, to display the data, store the data and compare the data to the stored data.

In the measuring device according to the invention the first signal preferably has a block-type signal shape. This signal shape is easy to generate using an electronic on/off switch.

In order to apply the measuring device according to the invention in industrial environments, the input and output electrical connection of the capacitive measuring circuit with identical components is protected against electrostatic discharge. Because of the use of identical components interference in the input voltage and output voltage, for instance phase shifts, is the same across the measuring circuit, and thereby not relevant for the calculation of the ratio.

The measuring device according to the invention preferably comprises 1,..., n measuring capacitors which are each plate-like, wherein each measuring capacitor is formed by a first and a second flat conductor having a non-conductive material therebetween, wherein the first conductor of each measuring capacitor is formed by a shared flat conductor, and the second conductors are arranged in a series on the non-conductive material, wherein the 1st, 3rd, 5th, etc. second conductors are connected electrically to each other, and the 2nd, 4th, 6th, etc. second conductors are connected electrically to each other. Each second conductor forms together with the first conductor a capacitor with a constant capacitance, while the 1st, 3rd, 5th, etc. second conductors and the 2nd, 4th, 6th, etc. second conductors together form a capacitor with a variable capacitance, this capacitance being dependent on the dielectric properties of the environment.

The invention will be further elucidated with reference to the following figures, in which:

Figure 1 shows a schematic overview of the preferred embodiment of the measuring device according to the invention.
Figure 2A shows a measuring circuit for application in a measuring device according to Figure 1.
Figure 2B shows a top view of the measuring circuit according to Figure 2A.
Figure 3 shows a ratio R measured as a function of time using the measuring device according to the invention.

The same components are designated in different figures with the same reference numerals.

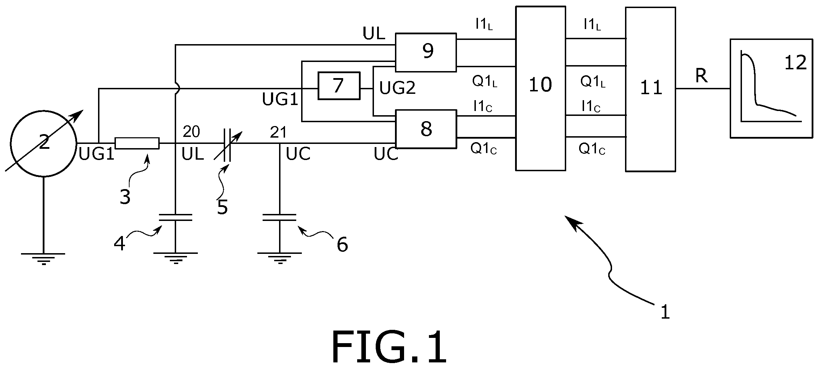

Figure 1 shows a schematic overview of the preferred a monument of measuring device 1 according to invention. Measuring device 1 is intended for the purpose of measuring change in the dielectric properties in an environment. Measuring device 1 comprises for this purpose a capacitive measuring circuit provided with an input and output electrical connection. The capacitive measuring circuit is formed by capacitors 4, 5 and 6, wherein the capacitance of capacitor 5 is variable and the capacitance of capacitors 4,6 is fixed. The capacitance of capacitor 5 can be influenced by the dielectric properties of the environment in which capacitor 5 is placed. Measuring device 1 further comprises an electric alternating signal generator 2 for applying a first electric alternating signal UG1 with a specific base frequency and a specific signal shape between the input and output connection of the measuring circuit. The signal UG1 is preferably a block wave with a frequency of 500-600 kHz.

The signal UG1 from signal generator 2 is further transmitted via a complex impedance as signal UL to the input connection of the measuring circuit. The measured signal UC can be determined at the output connection of the measuring circuit.

Measuring device 1 further comprises a first signal processing means 7 which shifts an input signal 90° in phase. Using first signal processing means 7 a second electric alternating signal UG2 is derived from the first signal UG1, wherein the second signal has the same base frequency and signal shape as the first signal UG2 and is shifted 90° in phase relative to the first signal UG1.

Measuring device 1 further comprises first quadrature determining means 8 for calculating the in-phase component $I1_C$ and quadrature component $Q1_C$ of an output voltage Uc across the output connection of the measuring circuit, wherein the first signal UG1 and second signal UG2 function as reference signals.

Measuring device 1 additionally comprises second quadrature determining means 9 for calculating the in-phase component $I1_L$ and quadrature component $Q1_L$ of an input voltage $U_L$ across the input connection of the measuring circuit, wherein the first signal UG1 and second signal UG2 function as reference signals.

Measuring device 1 comprises an analog to digital converter for converting the analog components ($I1_L$, $I1_C$, $Q1_L$, $Q1_C$) to a digital variant.

The digital components ($I1_L$, $I1_C$, $Q1_L$, $Q1_C$) are transmitted to a calculation circuit 11 configured to calculate a ratio R of the in-phase components ($I1_L$, $I1_C$) and quadrature components ($Q1_L$, $Q1_C$) of the output voltage ($U_L$) and input voltage ($U_C$) in accordance with the formula:

$$RATIO = \sqrt[2]{\frac{I1_L^2 + Q1_L^2}{I1_C^2 + Q1_C^2}}$$

The calculated ratio is subsequently transmitted to visual means 12 for displaying the calculated ratio R to a user of measuring system 1.

The calculation circuit can also be configured to calculate a differential quotient R' of the ratio R or an integral ∫ R of the ratio over a fixed time interval.

Visual means 12 preferably comprise storage means for storing a ratio R, differential of the ratio R' and/or integral ∫ R of the ratio in relation to time during a measurement, and comparing means for comparing a ratio to a stored ratio.

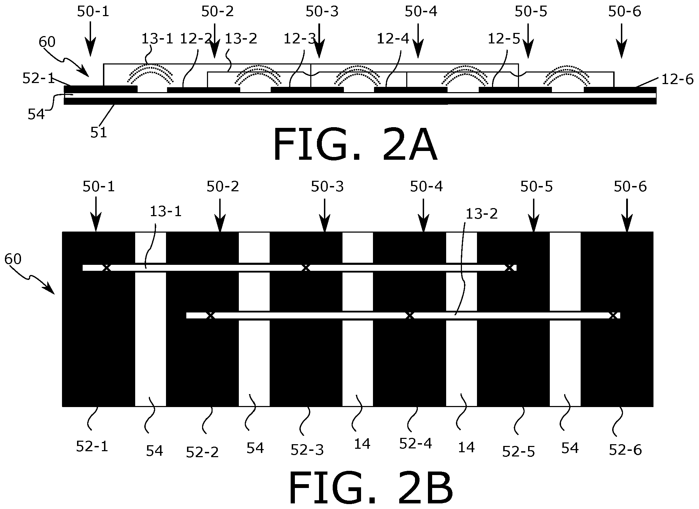

Figure 2A shows a measuring circuit 60 for application in a measuring device according to Figure 1, wherein measuring circuit 60 comprises measuring capacitors 50-1,..., 50-6, which are each plate-like. Each measuring capacitor 50-n is formed by a first shared conductor 54 and a second flat conductor 52-n with a non-conductive material 54 therebetween. The second conductors 52-1,..., 52-6 are arranged in a series on the non-conductive material 14. Conductors 52-1, 52-3 and 52-5 are connected electrically to each other by means of bridge 13-1. In addition, conductors 52-2, 52-4 and 52-6 are connected electrically to each other by means of bridge 53-2.

It will be apparent to the skilled person that conductor 51 and conductors 52-1,..., 52-6 form six separate capacitors.

However, the mutually adjacent conductors 52-1,..., 52-6 also form separate capacitors, the dielectric of which is formed by the environment around the conductors 52-n. The electrical field is indicated in the figure by means of the dotted curved lines.

As soon as the dielectric properties in the electric field have changed, the capacitance of the formed capacitor changes.

Figure 2B shows a top view of measuring circuit 60 according to Figure 2A. The strip-like conductors 52-1,..., 52-6 are shown more clearly in this figure.

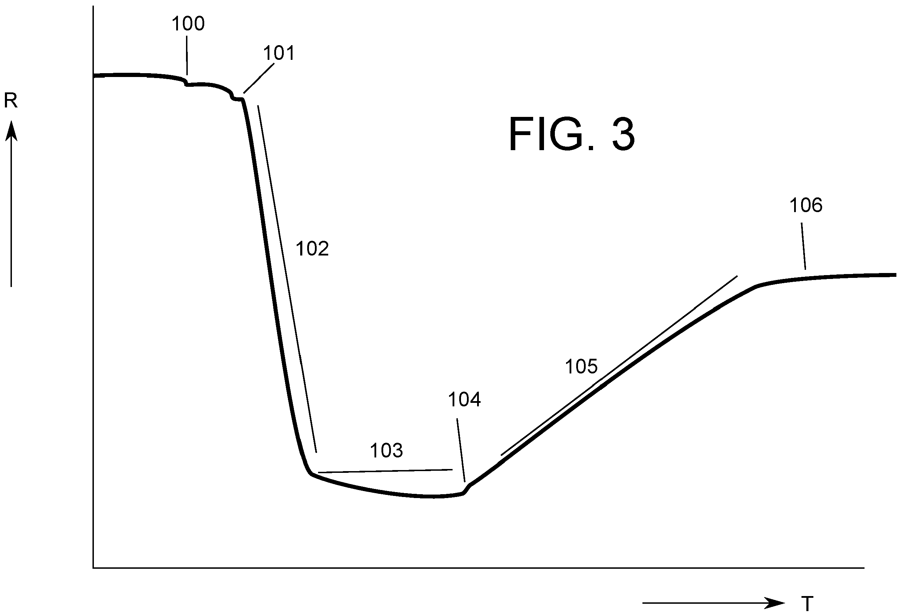

Figure 3 shows a measured ratio R as a function of time using the measuring device according to the invention. The measurement has taken place in a resin infusion moulding process as applied for instance in the manufacture of blades of wind turbines. The graph of R shows different moments which cannot be detected with known measuring means. The measurement shows the measured ratio R from a measuring device according to the invention, wherein the measuring circuit is arranged in the mould just below the contact surface between mould and the synthetic resin. The synthetic resin influences the capacitance of the measuring circuit in this process.

The graph shows the following moments:

    100 - compression of the materials arranged in the mould by means of vacuum
    101 - start of synthetic resin infusion
    102 - infusion speed
    103 - permeability
    104 - vacuum reduction (decompression)
    105 - cross-linking
    106 - approaching glass transition temperature (Tg).

The above moments in a resin infusion process cannot be determined with any other measuring method.

Most quality measurements for processes in plastic processing take place after the process and are usually destructive, this in contrast to the measuring device according to the invention, which can measure during the plastic processing and can be built in.

The measuring device according to the invention is so sensitive that the measuring circuit can be provided with a thin protective layer. The measuring circuit need not therefore come into direct contact with the environment to be measured. This is a significant drawback of current measuring devices, since measurement takes place during the process by placing the measuring circuit in the (casting) moulds.

By applying high-frequency techniques and ESD-protected electronics and low measuring impedance of the measuring device caused by the high frequency the measuring device according to the invention is able to determine with high accuracy the dielectric changes in a process and transmit these to subsequent systems. As a result the ratio of the change is calculated on the subsequent system and used in the process control of the subsequent system.

The invention is of course not limited to the described and shown preferred embodiment, but extends to any embodiment falling within the scope of protection as defined in the claims and as seen in the light of the foregoing description and accompanying drawings.

## Claims

1.  Measuring device (1) for measuring change in the dielectric properties in an environment, comprising

    - a capacitive measuring circuit provided with an input and output electrical connection (20;21), wherein the measuring circuit comprises one or more coupled measuring capacitors (5;50) which are placeable in the environment and wherein the capacitance of each of the measuring capacitors (5;50) can be influenced by the dielectric properties of the environment,
    - an electric alternating signal generator (2) connected a common ground for applying a first electric alternating signal with a specific base frequency and a specific signal shape to the input connection (20) of the measuring circuit wherein the input connection (20) and output connection (21) are indirectly connected to the common ground connection,

- a first signal processing means (7) for deriving a second electric alternating signal from the first signal, wherein the second signal has the same base frequency and signal shape as the first signal and is shifted 90° in phase relative to the first signal,

- first quadrature determining means (8) for calculating the in-phase component $I1_C$ and quadrature component Q1c of an output voltage Uc across the output connection (21) of the measuring circuit, wherein the first signal and second signal function as reference signals,

**characterized in that** the measuring device (1) is provided with

- second quadrature determining means (9) for calculating the in-phase component $I1_L$ and quadrature component $Q1_L$ of an input voltage $U_L$ across the input connection (20)of the measuring circuit, wherein the first signal and second signal function as reference signals,

- a calculation circuit (11) configured to calculate a ratio of the in-phase components ($I1_L$, $I1_C$) and quadrature components ($Q1_L$, $Q1_C$) of the output voltage ($U_L$) and input voltage ($U_C$).

2. Measuring device (1) as claimed in claim 1, wherein the following formula is determinant for calculating the ratio:

$$RATIO = \sqrt[2]{\frac{I1_L^2 + Q1_L^2}{I1_C^2 + Q1_C^2}}$$

3. Measuring device (1) as claimed in claim 1, wherein the signal generator (2) is provided with a second signal processing means for deriving a third electric alternating signal from the first signal, wherein the third signal has the same base frequency and signal shape as the first signal and is shifted 180° in phase relative to the first signal,

- a third signal processing means for deriving a fourth electric alternating signal from the first signal, wherein the fourth signal has the same base frequency and signal shape as the first signal and is shifted 270° in phase relative to the first signal,

- third quadrature determining means for calculating the in-phase component $I2_C$ and quadrature component $Q2_C$ of an output voltage Uc across the output connection (21) of the measuring circuit, wherein the third signal and fourth signal function as reference signals,

- fourth quadrature determining means for calculating the in-phase component $I2_L$ and quadrature component $Q2_L$ of an input voltage $U_L$ across the input connection 20 of the measuring circuit, wherein the third signal and fourth signal function as reference signals,

wherein the calculation circuit (11) is configured to calculate a ratio of the in-phase components ($I1_L$, $I2_L$, $11_C$, $I2_C$) and quadrature components ($Q1_L$, $Q2_L$, $Q1_C$, $Q2_C$) of the output voltage ($U_L$) and input voltage ($U_C$).

4. Measuring device (1) as claimed in claim 3, wherein the following formula is determinant for calculating the ratio:

$$RATIO = \sqrt[2]{\frac{(|I1_L|+|I2_L|)^2 + (|Q1_L|+|Q2_L|)^2}{(|I1_C|+|I2_C|)^2 + (|Q1_C|+|Q2_C|)^2}}$$

5. Measuring device (1) as claimed in any of the foregoing claims, wherein the calculation circuit (11) comprises one or more analog to digital converters for converting the analog in-phase signals and analog quadrature signals to digital in-phase and digital quadrature signals, and one or more programmed circuits for calculating the ratio with the digital in-phase and quadrature signals.

6. Measuring device (1) as claimed in any of the foregoing claims, wherein the calculation circuit (11) is also configured to calculate a differential quotient of the ratio.

7. Measuring device (1) as claimed in any of the foregoing claims, wherein the calculation circuit (11) is also configured to calculate an integral of the ratio over a fixed time interval.

8. Measuring device (1) as claimed in any of the foregoing claims, wherein the measuring device (1) comprises storage means for storing a ratio, differential of the ratio and/or integral of the ratio in relation to time during a measurement, and comparing means for comparing a ratio to a stored ratio.

9. Measuring device (1) as claimed in claim 8, to the extent dependent on claim 5, wherein the calculation circuit comprises a computer, wherein the computer is configured to calculate data relating to the ratio, the differential quotient of the ratio and/or the integral of the ratio, to display the data, store the data and compare the data to the stored data.

10. Measuring device (1) as claimed in any of the foregoing claims, wherein the first signal has a block-type signal shape.

11. Measuring device (1) as claimed in any of the foregoing claims, wherein the input and output electrical connection (20;21) of the capacitive measuring circuit with identical components is protected against electrostatic discharge.

12. Measuring device (1) as claimed in any of the foregoing claims, wherein the device (1) comprises 1,..., n measuring capacitors (50-1...50-6) which are each plate-like, wherein each measuring capacitor is formed by a first and a second flat conductor (51;52-1..52-6) having a non-conductive material (54) therebetween, wherein the first conductor (51) of each measuring capacitor is formed by a shared flat conductor, and the second conductors (52-1..52-6) are arranged in a series on the non-conductive material (54), wherein the 1st, 3rd, 5th, etc. second conductors (52-1;52-3;52-5) are connected electrically to each other, and the 2nd, 4th, 6th, etc. second conductors (52-2;52-4;52-6) are connected electrically to each other.

**Patentansprüche**

1. Messvorrichtung (1) zum Messen der Änderung der dielektrischen Eigenschaften in einer Umgebung, umfassend

- eine kapazitive Messschaltung, die mit einer elektrischen Eingangs- und Ausgangsverbindung (20; 21) versehen ist, wobei die Messschaltung einen oder mehrere gekoppelte Messkondensatoren (5; 50) umfasst, die in der Umgebung einsetzbar sind, und wobei die Kapazität jedes der Messkondensatoren (5; 50) durch die dielektrischen Eigenschaften der Umgebung beeinflusst werden kann,
- einen elektrischen Wechselstromgenerator (2), der mit einer gemeinsamen Masse verbunden ist, um ein erstes elektrisches Wechselsignal mit einer bestimmten Grundfrequenz und einer bestimmten Signalform an den Eingangsanschluss (20 des Messkreises anzulegen, wobei der Eingangsanschluss (20) und der Ausgangsanschluss (21) indirekt mit dem gemeinsamen Masseanschluss verbunden sind,
- ein erstes Signalverarbeitungsmittel (7) zum Ableiten eines zweiten elektrischen Wechselsignals aus dem ersten Signal, wobei das zweite Signal die gleiche Grundfrequenz und Signalform wie das erste Signal aufweist und um 90° in der Phase relativ zum ersten Signal verschoben ist,
- erste Quadraturbestimmungsmittel (8) zum Berechnen der phasengleichen Komponente $I1_C$ und der Quadraturkomponente $Q1_C$ einer Ausgangsspannung Uc über die Ausgangsverbindung der Messschaltung, wobei das erste Signal und das zweite Signal als Referenzsignale dienen,
**dadurch gekennzeichnet, dass** die Messvorrichtung (1) versehen ist mit
- zweiten Quadraturbestimmungsmitteln (9) zum Berechnen der Eingangsphasenkomponente $I1_L$ und der Quadraturkomponente $Q1_L$ einer Eingangsspannung $U_L$ über die Eingangsverbindung (20) der Messschaltung, wobei das erste Signal und das zweite Signal als Referenzsignale dienen,
- eine Berechnungsschaltung, die konfiguriert ist, um ein Verhältnis der Eingangsphasenkomponenten ($I1_L$, $I1_C$) und Quadraturkomponenten ($Q1_L$, $Q1_C$) der Ausgangsspannung ($U_c$) und der Eingangsspannung ($U_L$) zu berechnen.

2. Messvorrichtung (1) nach Anspruch 1, wobei die folgende Formel bestimmend für die Berechnung des Verhältnisses ist:

$$RATIO = \sqrt[2]{\frac{I1_L^2 + Q1_L^2}{I1_C^2 + Q1_C^2}}$$

3. Messvorrichtung (1) nach Anspruch 1, wobei der Signalgenerator (2) mit einem zweiten Signalverarbeitungsmittel zum Ableiten eines dritten elektrischen Wechselsignals aus dem ersten Signal versehen ist, wobei das dritte Signal die gleiche Grundfrequenz und Signalform wie das erste Signal aufweist und um 180° in der Phase gegenüber dem ersten Signal verschoben ist, **umfassend**

- ein drittes Signalverarbeitungsmittel zum Ableiten eines vierten elektrischen Wechselsignals aus dem ersten Signal, wobei das vierte Signal die gleiche Grundfrequenz und Signalform wie das erste Signal aufweist und um 270° in der Phase gegenüber dem ersten Signal verschoben ist,
- dritte Quadraturbestimmungsmittel zum Berechnen der Eingangsphasenkomponente $I2_C$ und der Quadraturkomponente $Q2_C$ einer Ausgangsspannung $U_C$ über die Ausgangsverbindung (21) der Messschaltung, wobei das dritte Signal und das vierte Signal als Referenzsignale dienen,
- vierte Quadraturbestimmungsmittel zum Berechnen der Eingangsphasenkomponente $I2_L$ und der Quadraturkomponente $Q2_L$ einer Eingangsspannung $U_L$ über die Eingangsverbindung (20) der Messschaltung, wobei das dritte Signal und das vierte Signal als Referenzsignale dienen,

wobei die Berechnungsschaltung (11) konfiguriert ist, um ein Verhältnis der Eingangsphasenkomponenten ($I1_L$, $I2_L$, $I1_C$, $I2_C$) und Quadraturkomponenten ($Q1_L$, $Q2_L$, $Q1_C$, $Q2_C$) der Ausgangsspannung ($U_C$) und der Eingangsspannung ($U_L$) zu berechnen.

4.  Messvorrichtung (1) nach Anspruch 3, wobei die folgende Formel determinierend für die Berechnung des Verhältnisses ist:

$$RATIO = \sqrt[2]{\frac{(|I1_L|+|I2_L|)^2 + (|Q1_L|+|Q2_L|)^2}{(|I1_C|+|I2_C|)^2 + (|Q1_C|+|Q2_C|)^2}}$$

5.  Messvorrichtung (1), wie in einem der vorstehenden Ansprüche beansprucht, wobei die Berechnungsschaltung (11) einen oder mehrere Analog-Digital-Wandler zum Umwandeln der analogen Eingangsphasensignale und analogen Quadratursignale in digitale Eingangsphasensignale und digitale Quadratursignale und eine oder mehrere programmierte Schaltungen zum Berechnen des Verhältnisses mit den digitalen Eingangsphasensignalen und Quadratursignalen umfasst.

6.  Messvorrichtung (1), wie in einem der vorstehenden Ansprüche beansprucht, wobei die Berechnungsschaltung (11) auch zum Berechnen eines Differentialquotienten des Verhältnisses konfiguriert ist.

7.  Messvorrichtung (1), wie in einem der vorstehenden Ansprüche beansprucht, wobei die Berechnungsschaltung (11) auch konfiguriert ist, um ein Integral des Verhältnisses über ein festes Zeitintervall zu berechnen.

8.  Messvorrichtung (1), wie in einem der vorstehenden Ansprüche beansprucht, wobei die Messvorrichtung (1) Speichermittel zum Speichern eines Verhältnisses, einer Differenz des Verhältnisses und/oder des Integrals des Verhältnisses in Bezug auf die Zeit während einer Messung und Vergleichsmittel zum Vergleichen eines Verhältnisses mit einem gespeicherten Verhältnis umfasst.

9.  Messvorrichtung (1) nach Anspruch 8, insoweit dieser von Anspruch 5 abhängig ist, wobei die Berechnungsschaltung einen Computer umfasst, wobei der Computer konfiguriert ist, um Daten in Bezug auf das Verhältnis, den Differentialquotienten des Verhältnisses und/oder das Integral des Verhältnisses zu berechnen, um die Daten anzuzeigen, die Daten zu speichern und die Daten mit den gespeicherten Daten zu vergleichen.

10.  Messvorrichtung (1), wie in einem der vorstehenden Ansprüche beansprucht, wobei das erste Signal eine blockartige Signalform aufweist.

11.  Messvorrichtung (1), wie in einem der vorstehenden Ansprüche beansprucht, wobei die elektrische Eingangs- und Ausgangsverbindung (20; 21) des kapazitiven Messkreises mit identischen Komponenten gegen elektrostatische Entladung geschützt ist.

12.  Messvorrichtung (1), wie in einem der vorstehenden Ansprüche beansprucht, wobei die Vorrichtung (1) 1,..., n Messkondensatoren (50-1...50-6) umfasst, die jeweils plattenförmig sind, wobei jeder Messkondensator durch einen ersten und einen zweiten Flachleiter (51; 52-1...52-6) mit einem dazwischen liegenden nicht leitenden Material (54) gebildet ist, wobei der erste Leiter jedes Messkondensators durch einen gemeinsamen Flachleiter gebildet ist und die zweiten Leiter (52-1...52-6) in einer Reihe auf dem nichtleitenden Material (54) angeordnet sind, wobei die ersten, dritten, fünften, usw. zweiten Leiter (52-1..52-3; 52-5) elektrisch miteinander verbunden sind, und die zweiten, vierten, sechsten, usw. zweiten Leiter (52,2; 52-4; 52-6) elektrisch miteinander verbunden sind.

## Revendications

1. Dispositif de mesure (1) pour mesurer un changement dans les propriétés diélectriques d'un environnement, comprenant

   - un circuit de mesure capacitif muni d'une connexion électrique d'entrée et de sortie (20 ; 21), le circuit de mesure comprenant un ou plusieurs condensateur(s) de mesure couplé(s) (5 ; 50) qui peuvent être placés dans l'environnement, et la capacité de chacun des condensateurs de mesure (5 ; 50) pouvant être influencée par les propriétés diélectriques de l'environnement,
   - un générateur de signal électrique alternatif (2) connecté à une masse commune pour appliquer à la connexion d'entrée (20) du circuit de mesure un premier signal électrique alternatif ayant une fréquence de base spécifique et une forme de signal spécifique, la connexion d'entrée (20) et la connexion de sortie (21) étant indirectement connectées à la connexion à la masse commune,
   - un premier moyen de traitement du signal (7) pour dériver un second signal électrique alternatif à partir du premier signal, le second signal ayant la même fréquence de base et la même forme de signal que le premier signal et étant déphasé de 90° par rapport au premier signal,
   - un premier moyen de détermination en quadrature de phase (8) pour calculer la composante en phase $I1_C$ et la composante en quadrature $Q1_C$ d'une tension de sortie UC sur la connexion de sortie (21) du circuit de mesure, le premier signal et le second signal servant de signaux de référence,
   **caractérisé en ce que** le dispositif de mesure (1) est muni
   - d'un second moyen de détermination en quadrature de phase (9) pour calculer la composante en phase $I1_L$ et la composante en quadrature $Q1_L$ d'une tension d'entrée $U_L$ sur la connexion d'entrée (20) du circuit de mesure, le premier signal et le second signal servant de signaux de référence,
   - d'un circuit de calcul (11) configuré pour calculer un ratio entre les composantes en phase ($I1_C$, $I1_L$) et les composantes en quadrature ($Q1_C$, $Q1_L$) de la tension de sortie ($U_c$) et la tension d'entrée ($U_L$).

2. Dispositif de mesure (1) selon la revendication 1, dans lequel la formule suivante est utilisée pour calculer le ratio :

$$RATIO = \sqrt[2]{\frac{I1_L^2 + Q1_L^2}{I1_C^2 + Q1_C^2}}$$

3. Dispositif de mesure (1) selon la revendication 1, dans lequel le générateur de signal (2) est muni d'un deuxième moyen de traitement du signal pour dériver un troisième signal électrique alternatif à partir du premier signal, le troisième signal ayant la même fréquence de base et la même forme de signal que le premier signal et étant déphasé de 180° par rapport au premier signal,

   - d'un troisième moyen de traitement du signal pour dériver un quatrième signal électrique alternatif à partir du premier signal, le quatrième signal ayant la même fréquence de base et la même forme de signal que le premier signal et étant déphasé de 270° par rapport au premier signal,
   - d'un troisième moyen de détermination de quadrature de phase pour calculer la composante en phase $I2_C$ et la composante en quadrature $Q2_C$ d'une tension de sortie UC sur la connexion de sortie (21) du circuit de mesure, le troisième signal et le quatrième signal servant de signaux de référence,
   - d'un quatrième moyen de détermination de quadrature de phase pour calculer la composante en phase $I2_L$ et la composante en quadrature $Q2_L$ d'une tension d'entrée $U_L$ sur la connexion d'entrée (20) du circuit de mesure, le troisième signal et le quatrième signal servant de signaux de référence,

   le circuit de calcul (11) étant configuré pour calculer un ratio entre les composantes en phase ($I1_L$, $I2_L$, $I1_C$, $I2_C$) et les composantes en quadrature ($Q1_L$, $Q2_L$, $Q1_C$, $Q2_C$) de la tension de sortie ($U_c$) et de la tension d'entrée ($U_L$).

**4.** Dispositif de mesure (1) selon la revendication 3, dans lequel la formule suivante est utilisée pour calculer le ratio :

$$RATIO = \sqrt[2]{\frac{(|I1_L|+|I2_L|)^2 + (|Q1_L|+|Q2_L|)^2}{(|I1_C|+|I2_C|)^2 + (|Q1_C|+|Q2_C|)^2}}$$

**5.** Dispositif de mesure (1) selon l'une quelconque des revendications précédentes, dans lequel le circuit de calcul (1) comprend un ou plusieurs convertisseurs analogique-numérique pour convertir les signaux en phase analogiques et les signaux en quadrature analogiques en signaux en phase numériques et en signaux en quadrature numériques, et un ou plusieurs circuits programmés pour calculer le ratio avec les signaux en phase et en quadrature numériques.

**6.** Dispositif de mesure (1) selon l'une quelconque des revendications précédentes, dans lequel le circuit de calcul (11) est également configuré pour calculer un quotient différentiel du ratio.

**7.** Dispositif de mesure (1) selon l'une quelconque des revendications précédentes, dans lequel le circuit de calcul (11) est également configuré pour calculer une intégrale du ratio sur un intervalle de temps fixe.

**8.** Dispositif de mesure (1) selon l'une quelconque des revendications précédentes, dans lequel le dispositif de mesure (1) comprend des moyens d'enregistrement pour enregistrer un ratio, une différentielle du ratio et/ou une intégrale du ratio par rapport au temps durant une mesure, et des moyens de comparaison pour comparer un ratio à un ratio enregistré.

**9.** Dispositif de mesure (1) selon la revendication 8, dans la mesure où elle dépend de la revendication 5, dans lequel le circuit de calcul comprend un ordinateur, l'ordinateur étant configuré pour calculer des données relatives au ratio, au quotient différentiel du ratio et/ou à l'intégrale du ratio, pour afficher les données, enregistrer les données et comparer les données aux données enregistrées.

**10.** Dispositif de mesure (1) selon l'une quelconque des revendications précédentes, dans lequel le premier signal a une forme de type signal de bloc.

**11.** Dispositif de mesure (1) selon l'une quelconque des revendications précédentes, dans lequel la connexion électrique d'entrée et de sortie (20 ; 21) du circuit de mesure capacitif ayant des composants identiques est protégée contre les décharges électrostatiques.

**12.** Dispositif de mesure (1) selon l'une quelconque des revendications précédentes, dans lequel le dispositif (1) comprend 1,..., n condensateurs de mesure (50-1 ... 50-6) qui sont chacun en forme de plaque, chaque condensateur de mesure étant formé par un premier et un second conducteur plat (51 ; 52-1 ... 52-6) avec entre eux un matériau non conducteur (54), le premier conducteur (51) de chaque condensateur de mesure étant constitué par un conducteur plat commun, et les seconds conducteurs (52-1 ... 52-6) étant disposés en série sur le matériau non conducteur (54), les 1er, 3ème, 5ème, etc. seconds conducteurs (52-1, 52-3, 52-5) étant connectés électriquement entre eux, et les 2nd, 4ème, 6ème, etc. seconds conducteurs (52-2, 52-4, 52-6) étant connectés électriquement entre eux.

FIG.1

FIG. 2A

FIG. 2B

FIG. 3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0922963 A

**Non-patent literature cited in the description**

- **HUAN L LEE.** *The Handbook of Dielectric Analysis and Cure Monitoring,* 2014